Europäisches Patentamt

European Patent Office

Office européen des brevets

(19)

(11) Numéro de publication : **0 083 284**
**B1**

(12) **FASCICULE DE BREVET EUROPÉEN**

(45) Date de publication du fascicule du brevet :
26.03.86

(21) Numéro de dépôt : 82402363.4

(22) Date de dépôt : 22.12.82

(51) Int. Cl.⁴ : **G 01 N 33/569, A 61 K 39/002**

(54) Système et procédé pour le dépistage de la toxoplasmose.

(30) Priorité : 24.12.81 FR 8124239

(43) Date de publication de la demande :
06.07.83 Bulletin 83/27

(45) Mention de la délivrance du brevet :
26.03.86 Bulletin 86/13

(84) Etats contractants désignés :
BE CH DE IT LI LU NL

(56) Documents cités :
FR-A- 1 602 301
US-A- 3 914 400
ANN. BIOL. CLIN., vol. 28, 1970, pages 411-415, Paris,
FR, P. COUZINEAU et al.: "Agglutination directe des
toxoplasmes"
SCIENCE, vol. 108, 10 décembre 1948, pages 660-
663 A.B. SABIN et al.: "Dyes as microchemical indicators of a new immunity phenomenon affecting a
protozoon parasite (toxoplasma)"
CHEMICAL ABSTRACTS, vol. 82, no. 13, 31 mars
1975, page 360, no. 84181p, Columbus, Ohio, US, Y.
PELOUX: "Direct agglutination reaction of toxoplasmas"

(73) Titulaire : **LABORATOIRES BIOTROL S.A.**
**1, rue du Foin**
**F-75140 Paris Cedex 03 (FR)**

(72) Inventeur : **Lawny, François**
**Lotissement Vieux Moulin**
**F-60350 Cuise-La-Motte (FR)**

(74) Mandataire : **Phélip, Bruno et al**
**c/o Cabinet Harlé & Phélip 21, rue de la Rochefou-**
**cauld**
**F-75009 Paris (FR)**

Jouve, 18, rue St-Denis, 75001 Paris, France

## Description

La présente invention concerne le dépistage de la toxoplasmose ; elle concerne plus particulièrement la détection de la toxoplasmose reposant sur une agglutination directe des toxoplasmes.

Parmi les tests connus de dépistage de la toxoplasmose, l'agglutination directe est un des plus répandus. Son principe est connu depuis 1965 ; il consiste à visualiser la présence éventuelle d'anticorps anti-toxoplasmes par l'agglutination d'une suspension de parasites dans une cupule de microtitrage. En présence d'anticorps, les parasites, reliés par un réseau de gammaglobulines, forment un voile qui tapisse le fond de la cupule. En l'absence d'anticorps, par contre, ils sédimentent en bouton. La technique correspondante est décrite par exemple dans « Agglutination Directe des Toxoplasmes » P. COUZINEAU et H. BAUFINE-DUCROCQ-Ann. Biol. Clin. 28, 411-415 (1970).

L'inconvénient majeur de cette réaction très spécifique est de fatiguer la vue des personnes qui effectuent ces tests. Les toxoplasmes sont pratiquement transparents et les voiles d'agglutination sont pratiquement invisibles. Les boutons de sédimentation exigent un éclairage rasant pour être bien visibles.

De ce fait, nombre de biologistes préfèrent utiliser des hématies sensibilisées avec un extrait antigénique de toxoplasmes. L'hémagglutination — ou la sédimentation — peut alors être constatée sans aucune fatigue visuelle. Cependant, pour être spécifique, la réaction nécessite qu'on épuise au préalable les sérums en anticorps anti-hématies, de façon à ne laisser subsister que les anticorps anti-toxoplasmoses. Cette technique présente également un autre inconvénient : comme elle fait appel à un antigène figuré, réalisé en greffant chimiquement une fraction antigénique solubilisée sur une hématie, la réponse de la réaction d'hémagglutination est moins parallèle à la technique de référence (dite « dyestest ») que l'agglutination directe.

La présente invention a pour but de procurer un moyen de dépistage de la toxoplasmose qui allie la spécificité à la lisibilité et permette ainsi de procéder à des tests sûrs, sans fatigue visuelle excessive.

On y parvient selon l'invention en facilitant la préparation du test et en rendant l'agglutination directe très facilement lisible grâce à la mise en œuvre d'un tampon de dilution coloré, c'est-à-dire d'un tampon de dilution auquel on a incorporé une substance ou un système procurant à ce tampon une coloration dans la gamme du spectre visible.

On a certes déjà décrit dans la demande de brevet EP-A-0 072 319 du 5 août 1981 au nom de la même demanderesse l'utilisation de toxoplasmes colorés, préparés en suspension stable dans un milieu liquide, pour le dépistage de la toxoplasmose. Cependant, avant même la mise en œuvre des toxoplasmes dans un test d'agglutination directe et la lecture des résultats, la préparation des puits ou cupules de microtitrage requiert une attention soutenue de la part du technicien qui l'effectue.

La réalisation d'un test d'agglutination directe comprend comme étape essentielle la préparation d'une plaque de microtitrage, qui se fait en trois temps :

1er temps : répartition dans tous les puits d'une plaque de microtitrage du tampon de dilution (milieu liquide),

2e temps : dilution de sérum selon une série de raison 1/2 dans ces puits de microtitrage.

3e temps : addition d'une suspension en milieu liquide d'antigènes, qui permettent de visualiser la présence ou l'absence d'anticorps anti-toxoplasmose par formation d'un voile d'agglutination ou d'un bouton de sédimentation sur le fond de la cupule de microtitrage.

Les milieux sont des milieux liquides, entièrement aqueux ou à base d'eau et d'au moins un autre solvant, tel que du glycérol ou autres.

On a maintenant trouvé qu'on peut améliorer sur plusieurs plans les tests de détection de la toxoplasmose aussi bien par agglutination directe conventionnelle, c'est-à-dire sans aucune coloration spéciale du système, que par la technique d'agglutination directe avec coloration des toxoplasmes décrite dans la demande de brevet susdit, en incorporant au tampon de dilution introduit dans les cupules de microtitrage dans le premier temps de ce procédé une substance ou un système procurant à ce tampon une coloration, avantageusement mais non exclusivement dans la gamme du spectre visible.

La présente invention a pour premier objet un système ou un ensemble pour la détection de la toxoplasmose par agglutination directe, comprenant un tampon de dilution et une suspension de toxoplasmes, caractérisé en ce que le tampon de dilution comprend au moins une substance colorée qui a le pouvoir de s'adsorber sur lesdits toxoplasmes, sans modifier leurs caractéristiques antigéniques et leur capacité d'agglutination en présence d'anticorps antitoxoplasmes.

Le colorant ou le système de coloration (qui peut, par exemple, comprendre un précurseur de colorant et un révélateur de ce dernier) — dénommé dans tous les cas ci-après pour simplifier « colorant » — peut être choisi par l'homme de métier parmi les colorants ou mélanges de colorants connus au moment où il a à les utiliser, après un simple test de compatibilité.

Avant de mettre en œuvre un colorant déterminé conformément à l'invention, il convient si cela n'a pas déjà été fait, de vérifier que par un test préalable si ce colorant est approprié ou non, le test à effectuer est double et peut se définir comme suit :

le colorant ne doit pas fausser l'analyse en induisant de fausses sédimentations ou une fausse agglutination ; on doit obtenir en sa présence et sans colorant le même titre d'agglutination pour le même sérum positif et les sérums négatifs ne doivent pas donner d'agglutination en présence de ce colorant ;

le colorant ne doit pas réagir avec les anticorps anti-toxoplasmes du sérum à doser.

En variante, le système ainsi défini peut également comporter une coloration des toxoplasmes conformément à la demande de brevet EP-A-0 072 319 du 5 août 1981 au nom de la même demanderesse, intitulée « Procédé amélioré pour le dépistage de la toxoplasmose et toxoplasmes colorés en suspension stable » et à laquelle il convient de se référer pour plus de détail à ce propos.

L'invention a donc également pour objet un système ou un ensemble pour la détection de la toxoplasmose par agglutination directe, tel que défini plus haut, caractérisé en ce que les toxoplasmes comprennent en outre eux-mêmes un colorant.

Ce colorant des toxoplasmes doit également satisfaire aux tests susdits.

Pour tirer le meilleur profit de cette variante, il est judicieux de recourir à des colorants sensiblement différents, et avantageusement complémentaires, respectivement pour le tampon et pour l'antigène.

Dans la technique d'agglutination directe, l'utilisation d'un tampon de dilution coloré selon l'invention facilite grandement le travail du technicien ; celui-ci peut ainsi savoir immédiatement quels sont les puits garnis et ceux qui ne le sont pas, alors que cette constatation est assez malaisée avec des tampons non colorés. De plus, l'utilisation complémentaire de toxoplasmes colorés permet d'obtenir des images très contrastées : par exemple, toxoplasmes bleu marine avec un tampon coloré en rose.

L'invention a donc pour autre objet un procédé pour le dépistage de la toxoplasmose par agglutination directe, mettant en œuvre successivement la distribution dans des réceptacles appropriés d'un tampon de dilution, la dilution des sérums à tester à des concentrations selon une série de progression déterminée dans ce tampon, et l'addition d'une suspension de toxoplasmes, caractérisé en ce qu'on met en œuvre un tampon de dilution comportant un colorant comme défini plus haut.

En variante on peut également mettre en œuvre dans ce procédé des toxoplasmes qui sont en suspension stable en milieu liquide et qui sont eux-mêmes pourvus au préalable d'un colorant ou d'un système de coloration comme décrit dans la demande de brevet EP-A-0 072 319 susdite.

En variante, aussi bien dans le cas où les toxoplasmes sont eux-mêmes colorés que dans le cas où ils ne le sont pas, il est avantageux que le pH du milieu de mise en suspension des toxoplasmes soit différent de celui du tampon de dilution et que le colorant (au sens large défini plus haut) donne lieu à un virage, révélant une couleur ou modifiant la couleur initiale du colorant du tampon, dû au changement de pH du milieu après l'addition de la suspension de toxoplasmes. On ajoute ainsi à la visualisation des puits garnis une visualisation de ceux où des toxoplasmes ont été ajoutés.

On a cependant également constaté que, pour autant que le colorant du tampon de dilution soit convenablement choisi, ce colorant est lui-même susceptible de s'adsorber sur des toxoplasmes non colorés et, donc, de fournir une image très contrastée des toxoplasmes colorés (ou foncés) dans un tampon clair, voire devenu incolore.

Cette opération d'adsorption est suffisamment rapide pour se dérouler pendant le laps de temps classiquement nécessaire à la réaction d'agglutination directe, c'est-à-dire en quelques heures à température ambiante. Les toxoplasmes non préalablement colorés sédimentent ainsi en quelques heures en adsorbant partiellement ou totalement le colorant du tampon.

Tout se passe comme si l'on avait utilisé des antigènes colorés, alors que l'on s'est évité la peine de les préparer.

Cela représente une variante tout particulièrement préférée de la présente invention.

L'invention a donc également pour objet un système ou un ensemble pour la détection de la toxoplasmose par agglutination directe, comprenant un tampon de dilution et une suspension d'antigènes ou toxoplasmes, caractérisé en ce que le tampon de dilution comprend une substance ou un système de coloration apte à s'adsorber, dans les conditions de la réaction d'agglutination, partiellement ou totalement sur les toxoplasmes et procurant à ceux-ci une coloration.

Là encore, avant de mettre en œuvre un tel colorant, il convient, si cela n'a pas déjà été fait, de s'assurer par un test préalable si ce colorant, choisi parmi ceux dont l'homme de métier peut disposer, est approprié ou non.

Le test à effectuer dans ce cas comprend le test double détaillé plus haut, mais ajoute à celui-ci les deux conditions suivantes :

le colorant doit être suffisamment adsorbé par l'antigène pour permettre un bon contraste de lecture,

le colorant ne doit pas modifier, par cette adsorption, les caractéristiques antigéniques des toxoplasmes.

Si ces deux critères, ainsi que les deux précédents, sont satisfaits, le colorant testé peut être retenu.

Des exemples de substances colorantes qui peuvent ainsi être mises en œuvre sont :

d'une manière générale tous les colorants des protéines, des lipides, des lipo-protéines, des mucopolysaccharides, des glycoprotéines et/ou des acides nucléiques,

plus particulièrement tous les colorants du cytoplasme, des vacuoles, du noyau et autres organites intracellulaires ou des membranes cellulaires ; et

plus spécifiquement, mais à titre d'exemples concrets uniquement :

le Noir Soudan B (qui est actuellement le colorant qu'on préfère à tous les autres), le Bleu Evans, le Vert Malachite.

Un autre objet encore de l'invention est par conséquent un procédé pour la réalisation d'essais de détection de la toxoplasmose par agglutination directe, mettant en œuvre successivement la distribution dans des réceptacles appropriés d'un tampon de dilution, la dilution des sérums à tester à des concentrations selon une série de progression déterminée dans ce tampon, et l'addition d'une suspension de toxoplasmes, caractérisé en ce qu'on met en œuvre un tampon de dilution comportant un colorant ou un système de coloration susceptible de s'adsorber sur les toxoplasmes dans les conditions de réalisation de l'essai d'agglutination directe et on effectue la lecture après qu'une adsorption partielle ou totale du colorant initialement contenu dans le tampon de dilution sur les toxoplasmes est intervenue.

L'invention a également pour objet des coffrets de détection de la toxoplasmose renfermant un tampon coloré et des toxoplasmes, eux-mêmes éventuellement colorés, qui sont tels que décrits plus haut.

Les exemples concrets ci-après sont donnés à titre d'illustration de l'invention, mais ne limitent en aucune manière celle-ci.

Exemple 1

On a préparé un tampon composé d'une solution aqueuse de :

| | |
|---|---|
| NaCl | 7,012 g/l |
| HBO$_3$ | 3,092 g/l |
| NaN$_3$ | 1 g/l |
| NaOH en pastilles | q.s.p. pH 9,0 |
| Albumine bovine | 4 g/l |

A ce tampon, on a ajouté de l'éosine en poudre à raison d'une concentration comprise entre 25 et 250 mg/l de tampon.

Sur un sérum à tester du point de vue de la présence éventuelle d'une infection par toxoplasmose, on a réalisé un test d'agglutination directe, suivant les règles de l'art, avec des toxoplasmes colorés en bleu marine par du Noir Soudan B.

La coloration du tampon permettait de savoir immédiatement quels puits avaient bien été remplis et lesquels ne l'étaient pas.

L'image obtenue était bien contrastée et n'était pas perturbée par rapport à une réaction témoin sur le même sérum aux mêmes dilutions, sans colorant du tampon.

Exemple 2

On a opéré comme dans l'exemple 1, mais en utilisant des toxoplasmes non colorés.

On a obtenu les mêmes avantages et l'image obtenue n'était pas perturbée par rapport à une réaction témoin sans colorant du tampon, faite sur le même sérum.

Exemple 3

On a reproduit le mode opératoire de l'exemple 1, mais en testant, comme colorant du tampon, du cristal violet à 2 g/l.

On a constaté de fausses agglutinations par comparaison avec un essai témoin.

Ce colorant doit donc être écarté.

Exemple 4

A un tampon préparé comme indiqué dans l'exemple 1, on a ajouté du Noir Soudan B en poudre à une concentration comprise entre 0,5 et 5 g/l de tampon.

On a réalisé un test d'agglutination directe avec une suspension de toxoplasmes non colorés.

Quatre heures plus tard, l'antigène avait sédimenté et on a constaté qu'il avait adsorbé une grande partie du colorant initial ; le tampon était en effet beaucoup plus clair et, au contraire, les toxoplasmes apparaissaient en bleu marine.

Après 16 heures, la décoloration du tampon et la coloration de l'antigène étaient maximales.

Le test d'agglutination directe effectué sur ce système ne donnait pas de résultats diférents d'une réaction témoin réalisée sans colorant.

La lisibilité de la plaque était très supérieure à celle de la plaque témoin, ce qui confère une sécurité accrue dans le résultat.

**Revendications**

1. Système pour la détection de la toxoplasmose par agglutination directe, comprenant un tampon de dilution et une suspension de toxoplasmes, caractérisé en ce que le tampon de dilution contient au moins une substance colorée qui a le pouvoir de s'adsorber sur lesdits toxoplasmes, sans modifier leurs caractéristiques antigéniques et leur capacité d'agglutination en présence d'anticorps antitoxoplasmes.

2. Système selon la revendication 1, caractérisé en ce que le colorant est choisi parmi les colorants des protéines, lipo-protéines, mucopolysaccharides, glycoprotéines et acides nucléiques.

3. Système selon la revendication 1 caractérisé en ce que le colorant est un colorant des organites ou membranes cellulaires.

4. Système selon l'une des revendications 1 à 3 caractérisé en ce que les toxoplasmes comprennent eux-mêmes un colorant compatible avec le colorant du tampon.

5. Système selon l'une des revendications 1 à 4, caractérisé en ce que le colorant du tampon est choisi parmi le Noir Soudan B, le Bleu Evans, le Vert Malachite et l'éosine.

6. Procédé d'agglutination directe pour le dépistage de la toxoplasmose, caractérisé en ce qu'il comprend la mise en œuvre d'un système selon l'une quelconque des revendications 1 à 5.

**Claims**

1. System for detecting toxoplasmosis by direct agglutination, comprising a dilution buffer and a suspension of toxoplasms, characterized in that the dilution buffer contains at least one coloured substance which has the ability to be adsorbed onto the said toxoplasms without modifying their antigenic characteristics and their capacity for agglutination in the presence of anti-toxoplasm antibodies.

2. System according to claim 1, characterized in that the colouring matter is chosen from stains for proteins, for lipoproteins, for mucopolysaccharides, for glycoproteins and for nucleic acids.

3. System according to claim 1, characterized in that the colouring matter is a stain for the cell organelles or membranes.

4. System according to one of claims 1 to 3, characterized in that the toxoplasms themselves contain a dye which is compatible with the colouring matter in the buffer.

5. System according to one of claims 1 to 4, characterized in that the colouring matter in the buffer is chosen from Sudan Black B, Evans Blue, Malachite Green and eosin.

6. Direct agglutination process for the detection of toxoplasmosis, characterized in that it comprises the use of a system according to any one of claims 1 to 5.

**Patentansprüche**

1. System zur Feststellung der Toxoplasmose durch direkte Agglutination, das eine Verdünnungspuffersubstanz und eine Toxoplasmensuspension umfaßt, dadurch gekennzeichnet, daß die Verdünnungspuffersubstanz wenigstens eine gefärbte Substanz enthält, die das Adsorptionsvermögen für die Toxoplasmen aufweist, ohne ihre antigenen Eigenschaften und ihre Agglutinationsfähigkeit in Gegenwart von antitoxoplasmischen Antikörpern zu ändern.

2. System nach Anspruch 1, dadurch gekennzeichnet, daß der Farbstoff unter den Farbstoffen der Proteine, der Lipoproteine, der Mukopolysaccharide, der Glykoproteine und der Nukleinsäuren ausgewählt ist.

3. System nach Anspruch 1, dadurch gekennzeichnet, daß der Farbstoff ein Farbstoff aus zellularen organischen Stoffen (organitos) oder aus Zellenmembranen ist.

4. System nach einem der Ansprüche 1 bis 3, dadurch gekennzeichnet, daß die Toxoplasmen selbst einen Farbstoff enthalten, der mit dem Farbstoff der Puffersubstanz verträglich ist.

5. System nach einem der Ansprüche 1 bis 4, dadurch gekennzeichnet, daß der Farbstoff der Puffersubstanz aus Sudan-schwarz B, Evansblau, Malachit-grün und Eosin ausgewählt ist.

6. Verfahren zur direkten Agglutination zwecks Feststellung von Toxoplasmose, gekennzeichnet durch die Anwendung eines Systems nach einem der Ansprüche 1 bis 5.